# EUROPÄISCHE PATENTANMELDUNG

(11) **EP 1 052 498 A1**
(43) Veröffentlichungstag der Anmeldung: **15.11.2000**
(21) Anmeldenummer: 00810332.7
(22) Anmeldetag: 17.04.2000
(51) Int. Cl.: G01N 13/00, G01N 33/15

(54) **Verfahren, Patrone und Vorrichtung zum Einbringen eines teilchenförmigen Materials in eine Flüssigkeit**

(30) Priorität: 11.05.1999 CH 89299
(71) Anmelder: SOTAX AG, CH-4123 Allschwil (CH)
(72) Erfinder: Benz, Rolf Martin, 4123 Allschwil (CH)
(74) Vertreter: Eder, Carl E.

(57) **Zusammenfassung**

Die Erfindung betrifft ein Verfahren zum Einbringen eines teilchenförmigen Materials (3), zum Beispiel von Pellets, von Granulat oder von Pulver, in eine Flüssigkeit sowie eine Patrone und eine Vorrichtung zur Durchführung dieses Verfahrens. Das Material (3) wird in eine Patrone mit einer Hülse (1), einer Achse (1c) und einem im Innern der Hülse (1) angeordneten und entlang der Achse (1c) von einem Anschlag (1d) weg verschiebbaren Boden (2) eingebracht. Anschliessend wird die Patrone in einer Position, in der ihre Achse (1c) ungefähr lotrecht ist und der Boden (2) infolge seines Gewichts am Anschlag (1d) ansteht, in die Flüssigkeit fallengelassen, wobei die Patrone durch ein Führungsrohr geführt wird. Beim Eintauchen in die Flüssigkeit wird der Boden (2) durch die Flüssigkeit vom Anschlag (1d) weg nach oben verschoben, wodurch das Material (3) mindestens zum grössten Teil aus der Patrone ausgeworfen wird.

## Beschreibung

Die Erfindung betrifft ein Verfahren zum Einbringen eines teilchenförmigen Materials, zum Beispiel von Pellets, von Granulat oder von Pulver, in eine Flüssigkeit.

Bei der Entwicklung und besonders bei der routinemässigen Qualitätskontrolle von festen Arzneimittelformen ist die Bestimmung der Wirkstofffreisetzungsgeschwindigkeit im Auflösungsprozess eines Arzneimittels von grosser Bedeutung. Aus diesem Grunde sind in den nationalen Arzneimittelbüchern und in der Europäischen Pharmakopoe genaue Vorschriften enthalten über die apparativen Parameter des Testgerätes wie auch über die Testdurchführung. Im Vordergrund steht dabei die United States Pharmakopoe (USP). Jedes Auflösungsprüfgerät muss zwingend den USP-Vorschriften entsprechen. Regelmässige Inspektionen der Gesundheitsbehörden, wie beispielsweise der Food and Drug Administration (FDA) der USA, kontrollieren strikte die Einhaltung der Vorschriften.

Gemäss den USP-Vorschriften erfolgt die Bestimmung der Wirkstofffreisetzung gleichzeitig mit sechs Prüflingen, die in je ein Prüfgefäss mit künstlichem Magen- oder Darmsaft gegeben werden. Der Auflösungsprozess erfolgt beispielsweise bei 37 Grad Celsius, wobei das Auflösungsmedium mittels eines standardisierten Rührers bei einer vorgegebenen Drehzahl gerührt wird. In vielen Fällen werden diese Prüfungen mit Tabletten oder Kapseln durchgeführt.

Ein Problem ergibt sich bei der Durchführung eines solchen Tests bei der Anwendung beispielsweise auf Medikamente, die als feines teilchenförmiges Material, zum Beispiel in der Form von Pellets, in Kapseln verabreicht werden. Pellets sind annähernd kugelförmige Teilchen, die typischerweise einen Durchmesser von 0,4 bis 1,2 mm, aufweisen. Aufgrund ihrer Grösse und ihrer Zusammensetzung neigen sie dazu, leicht elektrostatisch aufladbar zu sein. Wenn die Pellets nun automatisch, beispielsweise durch ein Metallröhrchen in die Flüssigkeit eingeleitet werden sollen, bleiben sie deshalb gerne an der Röhrchenwand haften. Ausserdem tendieren sie dazu, wegen der Oberflächenspannung der Flüssigkeit auf deren Oberfläche zu bleiben und nicht in die Flüssigkeit einzutauchen. Ein wesentliches Problem stellt zudem die Benetzbarkeit der Pellets, Pulver oder Granulate dar. Die Benetzbarkeit des zu prüfenden Materials bildet jedoch eine wichtige Voraussetzung für den reproduzierbaren Test. Aus diesen Gründen werden bisher bei Auflösungstests Pellets häufig auf einen Löffel abgewogen und mit diesem manuell in die Flüssigkeit eingerührt, worauf der Löffel wieder gereinigt werden muss. Das Verfahren ist deshalb natürlich mit einem erheblichen Zeit- und Kostenaufwand verbunden.

Der vorliegenden Erfindung liegt daher die Aufgabe zu Grunde, ein Verfahren zur Verfügung zu stellen, das das manuelle, halbautomatische oder automatische Einbringen von feinem teilchenförmigem Material, zum Beispiel von Pellets, von Granulat oder von Pulver in eine Flüssigkeit erlaubt, so dass die Dosierung mit ausreichender Genauigkeit kontrolliert werden kann, die gesamte vorgesehene Menge möglichst gleichzeitig zu einem definierten Zeitpunkt in die Flüssigkeit eingebracht werden kann und gleichzeitig eine gute Verteilung und damit eine möglichst gute Benetzung des Materials in der Flüssigkeit gewährleistet ist.

Diese Aufgabe wird gelöst durch ein Verfahren, wie es im Anspruch 1 definiert ist. Ebenfalls Gegenstand der vorliegenden Erfindung ist eine Patrone, die zur Durchführung dieses Verfahrens benutzt werden kann und die im Anspruch 6 aufgelisteten Merkmale besitzt, sowie eine Vorrichtung zur Durchführung des Verfahrens mit den Merkmalen des Anspruchs 10.

Vorteilhafte Weiterbildungen des Verfahrens, der Patrone und der Vorrichtungen gehen aus den abhängigen Ansprüchen hervor.

Das erfindungsgemässe Verfahren, die erfindungsgemässe Patrone und die erfindungsgemässe Vorrichtung weisen verschiedene Vorteile im Vergleich zum Stand der Technik auf. So ergibt die Erfindung den Vorteil, dass genau eine vorgegebene Menge teilchenförmiges Material auch bei kleiner Teilchengrösse problemlos in die Flüssigkeit eingebracht werden kann, ohne das Teilchen an irgend einem Vorrichtungsteil ausserhalb der Flüssigkeit haften bleiben oder dass sie auf der Oberfläche der Flüssigkeit verbleiben. Deshalb sind das erfindungsgemässe Verfahren, die Patrone und die Vorrichtung insbesondere auch gut geeignet zur Verwendung bei einer automatischen oder halbautomatischen Löslichkeitsprüfung, beispielsweise bei Qualitätskontrollen. Auch eignen sich Verfahren, Patrone und Vorrichtung speziell zum Einbringen von teilchenförmigen Materialien aus sehr kleinen Teilchen in eine Flüssigkeit, d.h. Materialien aus Teilchen mit einer Teilchengrösse von üblicherweise höchstens 1,2 mm und meistens mindestens 0,4 mm oder aus noch kleineren Teilchen.

Im Folgenden werden Ausführungsbeispiele der Erfindung anhand einer Zeichnung beschrieben. In der Zeichnung zeigt
die Figur 1 einen Axialschnitt einer Patrone zur Durchführung des erfindungsgemässen Verfahrens,
die Figur 2 eine Ansicht auf diese Patrone von oben,
die Figur 3 einen teilweisen Schnitt durch eine stark schematisierte Vorrichtung zur Durchführung des Verfahrens,
die Figur 4 eine Variante des Bodens der Patrone,
die Figur 5 eine weitere Variante des Bodens der Patrone und
die Figur 6 einen Axialschnitt einer anderen Ausführungsform der Patrone.

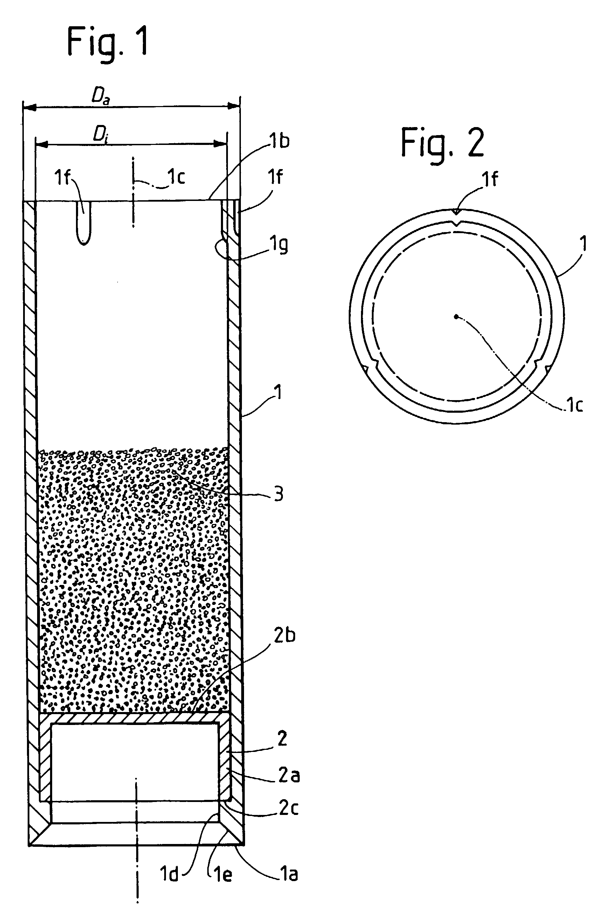

Eine Patrone zur Durchführung des erfindungsgemässen Verfahrens besteht aus einer im Wesentlichen zylindrischen Hülse 1 mit zwei Enden la und 1b und einem in der Hülse 1 verschiebbaren Boden 2. Sowohl Hülse 1 als auch Boden 2 bestehen aus einem gegen die Flüssigkeit resistenten Werkstoff, zum Beispiel aus rostfreiem Stahl. Die Hülse 1 ist als Hohlzylinder mit einem Aussendurchmesser *D*_{*a*} und einem Innendurchmesser *D*_{*i*} ausgebildet und besitzt eine Achse 1c. Sie weist nahe beim unteren Ende 1a eine als Anschlag ld dienende Verengung auf, wobei die Innenfläche auf der unteren Seite der Verengung beispielsweise noch einen sich zum unteren Ende la der Hülse 1 hin erweiternden konischen Abschnitt le aufweisen kann. Das andere Ende der Hülse ist beispielsweise mit drei kerbenförmigen Sicken lf versehen, die parallel zur Achse lc verlaufen und zusammen einen Sicherungsanschlag 1g bilden. Der Boden 2 befindet sich im Innern der Hülse 1. Im in der Figur 1 dargestellten Beispiel ist der Boden becherförmig, das heisst er besteht aus einem kurzen hohlzylindrischen Mantel 2a und einem plättchenförmigen, ungefähr ebenen Abschnitt 2b. Der Boden ist nun in die Hülse eingesetzt und mit kleinem Spiel in ihr verschiebbar geführt, so dass er mit nur minimaler Reibung entlang der Achse 1c zwischen dem Anschlag 1d und dem Sicherungsanschlag 1g gleiten kann und keine Teilchen des in die Patrone eingefüllten teilchenförmigen Materials 3 zwischen der Hülse 1 und dem Boden 2 herunterfallen können. In der aufrechten, im Folgenden Normalposition genannten Position, in der die Achse 1c der Hülse 1 lotrecht und der Boden 2 aufgrund seines Gewichtes unten ist, steht dabei der Rand 2c des Mantels 2b am Anschlag 1d an.

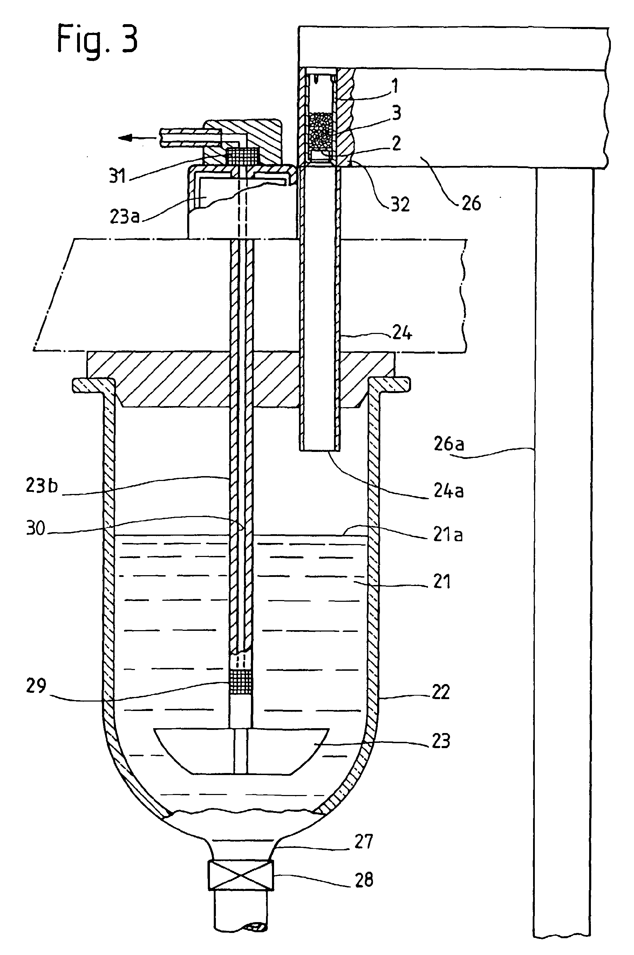

Als Nächstes wird kurz eine Vorrichtung zur Durchführung automatisierter Auflösungstests beschrieben. Eine solche Vorrichtung besitzt mehrere, beispielsweise sieben automatisch nachfüll- und reinigbare Flüssigkeitsbehälter 22. Diese Behälter können mit der gewünschten Flüssigkeit 21, beispielsweise mit künstlichem Magen- oder Darmsaft, gefüllt werden. An allen Behältern 22 bis auf einen können dann gleichzeitig Auflösungstests durchgeführt werden. Die Flüssigkeit 21 im übrigbleibenden Behälter 22 dient dabei als Referenz. Jeder Behälter 22 ist mit einer geeigneten und den Normen entsprechenden Rührvorrichtung versehen, also beispielsweise mit einem Paddel 23 mit einem motorischen Antrieb 23a. Für jeden Behälter 22, an dem Auflösungstests durchgeführt werden sollen, weist die Vorrichtung ein Führungsrohr 24 auf, durch das eine Patrone auf die Flüssigkeitsoberfläche 21a fallen gelassen werden kann. Jedes Führungsrohr 24 hat einen Innendurchmesser, der um ein wenig grösser ist als der Aussendurchmesser *D*_{*a*} einer Patrone, und eine Länge von mindestens einmal und beispielsweise ein- bis achtmal der Länge einer Patrone. Jedes Führungsrohr 24 ist über dem ihm zugeordneten Flüssigkeitsbehälter angebracht und ragt zum Beispiel von oben her etwas in diesen hinein und zwar so, dass die Achse des Führungsrohres lotrecht ist und dass das eine Ende 24a des Führungsrohres zum Beispiel mindestens eine halbe Patronenlänge und höchstens vier Patronenlängen über der Ebene ist, in der im Betriebszustand die Flüssigkeitsoberfläche 21a zu liegen kommt. Zusätzlich verfügt die Vorrichtung über eine in der Figur 3 lediglich schematisch dargestellte Zuführeinrichtung 26. Diese kann beispielsweise ein Magazin 32 mit einer geeigneten Anzahl Patronen besitzen, zum Beispiel ein Magazin mit 6 Reihen mit je 10 Patronen. Jeder Reihe ist dabei ein Führungsrohr zugeordnet, wobei beim Start eines Auflösungstests jeweils gleichzeitig aus jeder Reihe eine Patrone durch das der Reihe zugeordnete Führungsrohr 24 in den zugeordneten Flüssigkeitsbehälter 22 fallengelassen wird. Dabei wird das Magazin 32 jeweils zum gewünschten Zeitpunkt so verschoben, dass in ihm befindliche Patronen über das Führungsrohr 24 geschoben werden und folglich durch dieses in die Flüssigkeit 21 fallen. Die Einrichtung 26 und damit auch das Führungsrohr werden durch einen in der Figur 3 ebenfalls nur schematisch dargestellten Support 26a gehalten.

Die Vorrichtung besitzt im Weiteren noch Mittel und Einrichtungen zur Thermostatisierung der Flüssigkeitsbehälter 22 sowie zur intervallmässigen Probenentnahme aus den Behältern 22. Dazu kann beispielsweise die Flüssigkeit 21 durch einen Vorfilter 29, ein durch die Rührwelle 23b des Paddels 23 verlaufendes Rohr 30 und einen Filter 31 einer Messapparatur zugeführt werden, wo die Freisetzung der Teststoffe in der Flüssigkeit 21 beispielsweise photometrisch analysiert wird. Es ist aber auch ebensogut möglich, dass ein separates, zurückziehbares Rohr von oben her in die Flüssigkeit 21 eingeführt wird und die Probenentnahme durch dieses erfolgt. Damit auch Folgetests automatisiert durchgeführt werden können, verfügt die Vorrichtung über ein Befüll- Entleerungs- und Reinigunssystem für die Flüssigkeitsbehälter 22 sowie eine Einrichtung zur Entfernung leerer Patronen. Im gezeichneten Ausführungsbeispiel geschieht die Entleerung des Behälters 22 durch einen Auslass 27 mit einem Verschluss 28, durch den auch die Patrone zusammen mit der Flüssigkeit 21 abgeleitet werden kann. Ferner verfügt die Vorrichtung vorzugsweise noch über ein Steuerungsmittel, die das Befüllen der Flüssigkeitsbehälter 22, das Zuführen des Materials 3, die Probenentnahme, die Messungen, das Entleeren und die Reinigung der Behälter 22 zentral steuert, so dass eine Vielzahl von Tests automatisiert durchgeführt werden kann.

Zum Einbringen eines zu testenden teilchenförmigen löslichen Materials 3, im Folgenden Teststoff genannt, in eine als Testflüssigkeit dienende Flüssigkeit 21 wird die Hülse in einem ersten Schritt mit einer genau bemessenen Menge an Teststoff 3 gefüllt, beispielsweise mit einer vorgegebenen Masse wie zum Beispiel 400 mg. Die Menge kann zum Beispiel durch Wägen der Patrone und des in diese eingebrachten Materials oder durch Abwägen des Materials vor dem Einbringen in die Patrone oder eventuell durch eine volumetrische Messung festgelegt werden. Wie das in den Figuren 1 und 3 dargestellt ist, kann die Patrone gefüllt werden, wenn sie aufrecht, d.h. in der Normalposition ist. Die Hülse 1 dient dann zusammen mit dem Boden 2, der ausreichend dicht an der Innenfläche der Hülse 1 anliegt, als Gefäss. In einem nächsten Schritt wird die gefüllte Patrone als Ganzes aus der Normalposition auf die Flüssigkeitsoberfläche 21a fallengelassen. Die Patrone wird dabei durch ein Führungsrohr 24 geführt. Durch den Aufprall des Bodens und die entstehenden Auftriebskräfte beim Eintauchen der Patrone in die Flüssigkeit 21 wird dann der Boden 2 in der Hülse 1 angehoben, bis an den Sicherungsanschlag 1g verschoben und durch diesen gegen ein Verlassen der Hülse 1 gesichert. Dabei wird der Teststoff 3 ausgeworfen. Es hat sich nun gezeigt, dass der Teststoff 3 grösstenteils in die Flüssigkeit 21 ausgeworfen und bereits unmittelbar nach dem Auswerfen allseits von der Flüssigkeit 21 umschlossen sind. Es verbleibt also höchstens ein kleiner Teil des Materials auf der Flüssigkeitsoberfläche 21a. Der Teststoff 3 kann daher in gut definierter, vorgesehener und reproduzierbarer Weise in die Flüssigkeit eingebracht und dann mit dem Paddel mit der Flüssigkeit vermischt werden. Die Patrone sinkt in der Folge auf den Boden des Flüssigkeitsbehälters 22 ab und bleibt dort liegen. Teststoffreste, die eventuell noch in der Patrone verblieben sind, gelangen rasch in Kontakt mit der Flüssigkeit 21 und können in der Folge noch aufgelöst werden.

Der Auftrieb, der nach dem Aufprall der Patrone auf der Flüssigkeitsoberfläche 21a bewirkt, dass der Boden 2 der Patrone angehoben und bis an den Sicherungsanschlag 1g verschoben wird, hat verschiedene Ursachen. Einerseits wird er natürlich dadurch erzeugt, dass durch das Eindringen der Patrone, in der der Boden am Anschlag ld ansteht, Flüssigkeit verdrängt wird, wogegen die Gewichtskraft und, beim schnellen Eintauchen, auch die Trägheit der Flüssigkeit entgegenwirkt. Andererseits wirkt aber auch der Boden 2 selber als Schwimmer, denn beim Eintauchen der Patrone bleibt unter dem Becher, der vom Boden 2 gebildet wird, ein Luftkissen zurück.

Je nach Höhe, aus der die Patrone auf die Flüssigkeitsoberfläche 21a fallen gelassen wird, kommt eventuell noch ein weiterer vorteilhafter Effekt dazu, der den Auswurf des teilchenförmigen Materials 3 aus der Patrone optimiert. Durch die Trägheit des Bodens 2 und des in die Patrone eingefüllten Materials 3 sowie der Flüssigkeit 21 wird die Luftglocke, die sich unter dem 2 Boden bildet, kurzzeitig und vorübergehend komprimiert, wodurch der Auswurf ein wenig verzögert erfolgt. Das bewirkt, dass das teilchenförmige Material 3 beispielsweise grösstenteils erst ausgeworfen wird, wenn die Patrone schon ganz in die Flüssigkeit 21 eingetaucht ist, wodurch der Anteil der auf der Oberfläche verbleibenden Pellets, Granulat- oder Pulverkörner weiter reduziert wird.

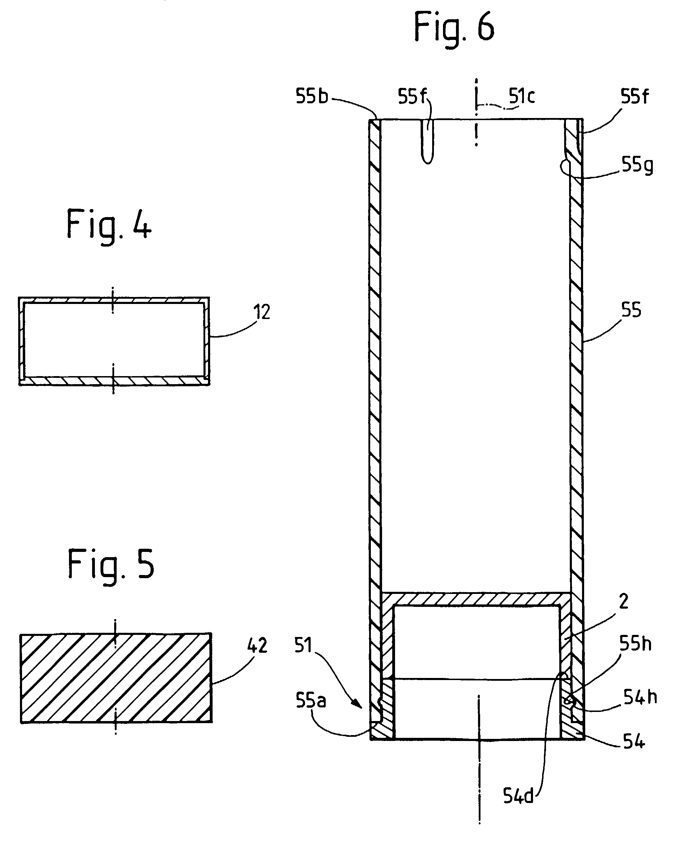

Das erfindungsgemässe Verfahren, die erfindungsgemässe Patrone und die erfindungsgemässe Vorrichtung können auch andere Ausgestaltungen aufweisen als die vorstehend beschriebene. Die Figur 4 zeigt beispielsweise eine andere Ausführungsform des Bodens 12. Der Boden 12 ist eine Dose mit einer allseitig geschlossenen Wandung aus metallischem Material, beispielsweise rostfreiem Stahl, die mit Luft gefüllt ist. Eine weiter Variante ist in der Figur 5 dargestellt. Der Boden ist ein Kunststoffkörper 42 aus einem leichten Kunststoff, der gegen die Testflüssigkeit resistent und/oder noch mit einem gegen die Testflüssigkeit resistenten Metallfilm beschichtet ist, beispielsweise mit einem Gold- oder Aluminiumfilm. Wesentlich bei diesen Ausführungsformen ist, dass die Dichte des Bodens als Ganzes kleiner ist als diejenige der Flüssigkeit 21, also beispielsweise kleiner als 1g/cm³, damit der Auftrieb des Bodens auch bei diesen Ausgestaltungen zur Aufwärtsverschiebung des Bodens beiträgt.

Wie in der Figur 6 aufgezeigt, kann auch die Hülse anders ausgestaltet sein als das in der Figur 1 dargestellte Beispiel und muss beispielsweise insbesondere nicht aus einem einstückigen Körper gefertigt sein. Die in der Figur 6 als Ganzes mit 51 bezeichnete Hülse setzt sich zusammen aus einem Hohlzylinder 55 mit einem unteren 55a und einem oberen Ende 55b und einem an dessen unterem Ende 55a befestigten ringförmigen Endstück 54. Der Hohlzylinder 55 kann beispielsweise aus Kunststoff angefertigt sein und weist am oberen Ende 55b ebenso wie die in der Figur 1 gezeichnete Ausführungsform Sicken 55f auf. Weiter besitzt der Hohlzylinder 55 noch in der Nähe seines unteren Endes 55a eine Ringnut 55h. Das Endstück 54 ist beispielsweise metallisch und weist einen ringsherumlaufenden Wulst 54h auf, der, wenn das Endstück 54 auf den Hohlzylinder 55 aufgesteckt ist, in die Ringnut 55h des Hohlzylinders eingreift. Der Wulst 54h und die Ringnut 55h können zum Beispiel zusammen ein Rastmittel bilden, so dass das Endstück 54 beim Zusammenbauen am Hohlzylinder 55 angesteckt und dabei angeklipst werden kann. In die H|lse 51 ist ein Boden 2 eingesetzt, der von der gleichen Ausführungsform ist wie der in der Figur 1 dargestellte Boden 2. Der Boden 2 ist in der Hülse 51 entlang ihrer Achse 51c zwischen einem Anschlag 54d und einem Sicherungsanschlag 55g verschiebbar, wobei der Anschlag 54d vom Endstück 54 und der Sicherungsanschlag 55g von den Sicken 55f gebildet wird. Es sei hier noch erwähnt, dass es selbstverständlich noch andere Möglichkeiten gibt ein Endstück, das einen Anschlag aufweist oder bildet, an einem Hohlzylinder anzubringen. So kann auch der Hohlzylinder 55 eine Wulst aufweisen, die dann in eine Ringnut im Endstück eingreift. Das Endstück kann auch als dünne Hülse ausgebildet sein, die aussen am Hohlzylinder angesteckt ist und einen an dessen unterem Ende 55a in die Innenseite des Zylinders ragenden Anschlag besitzt, in diesem Fall wird vorzugsweise das Endstück eine nach innen ragende und in eine Ringnut an der Aussenseite des Hohlzylinders eingreifende Wulst aufweisen. Auch ist es nicht notwendig, dass der Hohlzylinder 55 aus Kunststoff und das Endstück 54 aus Metall gefertigt ist, es kann ebensogut umgekehrt sein und es ist natürlich auch möglich, dass sowohl Hohlzylinder 55 als auch Endstück 54 aus Metall bzw. Kunststoff gefertigt sind.

Für die vorliegende Erfindung sind noch viele andere Ausführungsformen denkbar. So wäre es zum Beispiel ohne weiteres möglich, die Hülse 1 auch anders als zylindrisch, zum Beispiel im Querschnitt im Allgemeinen viereckig mit abgerundeten Ecken auszubilden. Auch der Boden muss durchaus nicht becher- oder dosenförmig sein, sondern kann beispielsweise auch als Kugel ausgebildet sein. Auch ist es nicht unbedingt notwendig, dass die Hülse 1 über einen Sicherungsanschlag lg verfügt. Es ist nämlich auch ohne weiteres denkbar, dass der Boden 2 beim Eintauchen der Patrone in die Flüssigkeit 21 aus der Patrone mit ausgeworfen wird. Der Boden kann dann später wieder eingesetzt werden. Wenn die Patrone genügend kostengünstig angefertigt werden kann, eignet sich diese Variante aber auch dazu, die Patrone als Einwegpatrone zu verwenden. Weiter muss die Vorrichtung zum Einbringen des teilchenförmigen Materials 3 nicht unbedingt ein Führungsrohr 24 aufweisen, die Patrone kann aus ihrer Normalposition automatisch oder manuell auch ungeführt auf die Flüssigkeitsoberfläche 21a fallen gelassen werden.

Das erfindungsgemässe Verfahren ist übrigens keineswegs beschränkt auf Anwendungen für Auflösungstests von pharmazeutischen Wirkstoffen im künstlichen Magen- oder Darmsaft. Es können ohne weiteres auch Auflösungstests mit Hilfsstoffen durchgeführt werden. Auflösungstests, in denen das erfindungsgemässe Verfahren, die erfindungsgemässe Patrone und die erfindungsgemässe Vorrichtung verwendet werden können, werden aber auch mit Nahrungsmitteln, mit Farbstoffen, mit Produkten der Agrochemie etc. durchgeführt, wobei die als Lösungsmittel dienende Flüssigkeit und ihre Temperatur natürlich jeweils entsprechend der Aufgabenstellung gewählt wird.

## Patentansprüche

1. Verfahren zum Einbringen eines teilchenförmigen Materials (3), zum Beispiel von Pellets und/oder von Granulat und/oder von Pulver, in eine Flüssigkeit (21), dadurch gekennzeichnet, dass das Material(3) in eine Patrone mit einer Hülse (1), einer Achse (1c) und einem im Innern der Hülse (1) angeordneten und entlang der Achse (lc) von einem Anschlag (1d) weg verschiebbaren Boden (2, 12) eingebracht wird, und dass die Patrone in einer Position, in der ihre Achse (1c) ungefähr lotrecht ist und der Boden (2, 12) infolge seines Gewichts am Anschlag (ld) ansteht, in die Flüssigkeit (21) fallengelassen wird, so dass der Boden (2, 12) durch die Flüssigkeit (21) vom Anschlag (1d) weg nach oben verschoben wird und das Material (3) mindestens zum grössten Teil aus der Patrone ausgeworfen wird.

2. Verfahren nach Anspruch 1, dadurch gekennzeichnet, dass eine Patrone verwendet wird, von der nach dem Einbringen in der Flüssigkeit (21) mindestens alle mit diesem in Kontakt kommenden Teile aus einem gegen die Flüssigkeit resistenten und in dieser unlöslichen Werkstoff, vorzugsweise einem metallischen Werkstoff, zum Beispiel rostfreiem Stahl, bestehen und dass die Patrone mindestens während einer gewissen Zeitdauer in der Flüssigkeit (21) belassen wird, so dass allenfalls in der Patrone verbliebenes Material (3) noch gelöst wird, wobei die Flüssigkeit (21) nach dem Einbringen der Patrone vorzugsweise gerührt wird.

3. Verfahren nach Anspruch 1 oder 2, dadurch gekennzeichnet, dass die Patrone beim Fallenlassen in einem vertikalen Führungsrohr (24) geführt wird.

4. Verfahren nach einem der Ansprüche 1 bis 3, dadurch gekennzeichnet, dass nach dem Einbringen des Materials (3) in die Flüssigkeit (21) die Auflösung des Materials (3) untersucht wird.

5. Verfahren nach einem der Ansprüche 1 bis 4, dadurch gekennzeichnet, dass mindestens eine Reihe von Patronen, wobei jeder Reihe ein Flüssigkeit (21) enthaltender Behälter (22) zugeordnet wird, so gehalten wird, dass durch einen Verschiebungsschritt einer jeden Reihe je eine einzelne Patrone in den der Reihe zugeordneten Behälter (22) fällt.

6. Patrone zur Durchführung des Verfahrens nach einem der Ansprüche 1 bis 5, dadurch gekennzeichnet, dass sie eine Hülse (1) mit einer Achse (1c) und einem Anschlag (ld) und einen verschiebbar in der Hülse (1) angeordneten, in einer Stellung der Patrone infolge seines Eigengewichtes am Anschlag (1d) anstehenden Boden (2, 12) aufweist.

7. Patrone nach Anspruch 6, dadurch gekennzeichnet, dass die Hülse (1) im Wesentlichen hohlzylinderförmig ist und ein unteres offenes Ende (la), ein oberes offenes Ende (1b) sowie zusätzlich zum genannten Anschlag (1d) noch einen in der Nähe des oberen Endes (1b) angeordneten Sicherungsanschlag (1g) aufweist, so dass der Boden (2, 12) zwischen dem Anschlag (ld) und dem Sicherungsanschlag (lg) verschiebbar ist.

8. Patrone nach Anspruch 6 oder 7, dadurch gekennzeichnet, dass der Boden (2) so ausgebildet und in die Hülse (1) eingesetzt ist, dass sich unter dem Boden (1) ein Luftkissen bildet, wenn die Patrone mit lotrechter Achse (1c) auf eine Flüssigkeitsoberfläche (21a) fallen gelassen wird.

9. Patrone nach einem der Ansprüche 6 bis 8, dadurch gekennzeichnet, dass der Boden (2) einen plattenförmigen Abschnitt (2b) und einen hülsenförmigen Mantel (2a) aufweist, der an seinem dem plattenförmigen Abschnitt (2b) abwegwandten Ende (2c) offen ist und vom plattenförmigen Abschnitt (2b) weg nach unten ragt, wenn sich die Patrone in einer Position befindet, in der ihre Achse (1c) lotrecht ist und der Boden (2) infolge seines Gewichts am Anschlag (1d) ansteht.

10. Vorrichtung zur Durchführung des Verfahrens nach einem der Ansprüche 1 bis 5, gekennzeichnet durch mindestens eine Patrone nach einem der Ansprüche 6 bis 9 und durch mindestens ein Führungsrohr (24) mit einer lotrechten Achse und einem unteren offenen Ende (24a) zum Führen einer in einen dem Führungsrohr (24) zugeordneten, Flüssigkeit (21) enthaltenden Behälter (22) fallenden Patrone.

11. Vorrichtung nach Anspruch 10, gekennzeichnet durch Mittel (26), die ein verschiebbar geführtes Magazin (32) zum Halten mindestens einer Reihe von Patronen aufweisen, wobei jeder Reihe ein Führungsrohr (24) zugeordnet ist, und dass die Mittel (26) ausgebildet sind, um das Magazin (32) schrittweise in Stellungen zu verschieben, in denen aus jeder Reihe eine Patrone durch das der Reihe zugeordnete Führungsrohr (24) in den dem Führungsrohr (24) zugeordneten Behälter (22) hinunterfällt.

12. Vorrichtung nach Anspruch 11, dadurch gekennzeichnet, dass mehrere Reihen von Patronen vorhanden sind, dass bei einem Verschiebungsschritt des Magazins im Wesentlichen gleichzeitig aus jeder Reihe eine Patrone hinunterfällt, und dass beispielsweise die Mittel (26) ausgebildet sind, um automatisch Flüssigkeit (21) in die Behälter(22) einzufüllen, die Patronen danach in die Flüssigkeit (21) fallenzulassen, danach eine Untersuchung der Auflösung des Materials (3) in der Flüssigkeit (21) zu bewirken und danach ein Abführen der Flüssigkeit aus den Behältern (22) und eine Reinigung der letzteren zu bewirken.
